# EUROPEAN PATENT APPLICATION

(11) **EP 2 322 133 A1**
(43) Date of publication of application: **18.05.2011**
(21) Application number: 09808276.1
(22) Date of filing: 19.08.2009
(51) Int. Cl.: A61K 6/00, A61L 27/00

(54) **METHOD FOR RESTORING MISSING TOOTH AND METHOD FOR PRODUCING RESTORATIVE MATERIAL**

(30) Priority: 20.08.2008 JP 2008211870
(71) Applicant: Organ Technologies Inc., Chiyoda-ku Tokyo 101-0048 (JP)
(72) Inventor: TSUJI, Takashi, Nagareyama-shi Chiba 270-0111 (JP); IKEDA, Etsuko, Ibaraki-shi Osaka 567-0846 (JP); ASAI, Hiroaki, Tokyo 101-0048 (JP)
(74) Representative: Goddar, Heinz J.
(86) International application number: PCT/JP2009/064509
(87) International publication number: WO 2010/021340

(57) **Abstract**

A method of producing a restorative material used to restore a tooth-deficient area in an oral cavity, the method comprising: positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other; culturing the first and second cell masses to form a reconstructed tooth germ or tooth; and confirming directionality of the reconstructed tooth germ or tooth formed by the culturing so as to enable the reconstructed tooth germ or tooth to be embedded in the tooth-deficient area such that a tip of the tooth faces an interior of the oral cavity, the tooth germ or tooth whose directionality has been confirmed being used as a restorative material to obtain an equivalent of a missing tooth in the tooth-deficient area. A method for restoring a tooth-deficient area, the method comprising; embedding the reconstructed tooth germ or tooth obtained by the production method in the tooth-deficient area.

## Description

### TECHNICAL FIELD

The present invention relates to a method for restoring a tooth-deficient area and a method for producing a restorative material.

### BACKGROUND ART

A tooth is an organ having: enamel in the outermost layer and dentin in its inner layer, both of which are hard tissues; odontoblasts, which produce the dentin, inside the dentin; and dental pulp in the central portion. Teeth may be lost by dental caries, periodontal diseases or the like, and from the perspective of the significant influence of the presence or absence of teeth on appearance and taste of food, and from the perspective of maintaining health and a high quality of life, various tooth regenerative techniques have been developed.

For example, J. Dent. Res., 2002, Viol.81(10), pp.695-700 discloses that, by intraperitoneally transplanting cells, such as epithelial cells or mesenchymal cells isolated from a tooth germ, to a mouse together with a biodegradative carrier, a tooth-like tissue is regenerated.
For example, as a method for regenerating a tooth germ, Japanese Patent Application Laid-Open (JP-A) No. 2004-331557 discloses a method in which tooth germ cells isolated from a living body are cultured in the presence of a physiologically active substance such as fibroblast growth factor. Further, JP 2004-357567 A proposes a method in which at least one type of cells selected from tooth germ cells isolated from a living body and cells capable of differentiating thereinto are cultured together with a carrier containing fibrin, wherein the carrier containing fibrin has a shape that allows a tooth germ to be formed in a shape of interest, thereby forming a "tooth" having a unique morphology.

WO 2006/129672 discloses a technique in which epithelial cells and mesenchymal cells derived from a tooth germ are made into cell masses, which are then placed in collagen gel such that these cell masses are closely contacting with each other, and the cell masses are cultured while being kept under such a condition, thereby producing a tooth having a cell arrangement unique to a tooth.

On the other hand, WO 2003/101503 discloses a method in which tooth germ cells and cells capable of differentiating thereinto are cultured under mechanical stimulation to regenerate a tooth germ, and a therapeutic method in which the thus-regenerated tooth germ is transplanted to the jawbone of a patient wherein a tooth germ is lost or damaged.
Further, JP 2005-013261 A discloses an artificial biomaterial having colloidal silica particles attached on its surface as an implant material to be used by being embedded in the living body.

However, it has been pointed out that implanting of a screw-shaped implant made of a material such as titanium in implant therapy at present suppresses growth of the jawbone during the growth period of jaw and makes tooth migration impossible, which are problematic. Further, although achievement of normal occlusion is necessary for a regenerated tooth to have the same function as that of a normal tooth, such occlusion has not been sufficiently confirmed in the above techniques using cells isolated from the living body. Further, nerves in the periodontal ligament have responsiveness to noxious stimuli of the tooth such as compression, and this is biologically important in view of perception such as feeling of food. For a regenerated tooth to have the same function as that of a normal tooth, the tooth-deficient area is expected to be restored such that the regenerated tooth allows normal occlusion to have hardness equivalent to that of a normal tooth and the nerves extend into the regenerated tooth whereby the tooth has normal responsiveness to stimulation as a tooth.

### DISCLOSURE OF THE INVENTION

### MEANS FOR SOLVING THE PROBLEMS

Accordingly, the object of the present invention is to provide: a method for producing a restorative material to restore a tooth-deficient area such that the regenerated tooth has hardness equivalent to that of a normal tooth, such that normal occlusion is attained, and such that the regenerated tooth has responsiveness to stimuli equivalent to that of a normal tooth; and a method for restoring a tooth-deficient area.

The present invention provides a method for producing a restorative material used for restoration of a tooth-deficient area in an oral cavity, and a method for restoring a tooth-deficient area.
The first aspect of the present invention provides a method of producing a restorative material used to restore a tooth-deficient area in an oral cavity, the method including:
positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other;
culturing the first and second cell masses to form a reconstructed tooth germ or tooth; and
confirming directionality of the reconstructed tooth germ or tooth formed by the culturing so as to enable the reconstructed tooth germ or tooth to be embedded such that the tip of the tooth faces the interior of the oral cavity; and,
the tooth germ or tooth whose directionality has been confirmed being used as a restorative material to obtain an equivalent of a missing tooth in the tooth-deficient area.

The second aspect of the present invention provides a method of restoring a tooth-deficient area in an oral cavity, the method including:
positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other;
culturing the first and second cell masses to form a reconstructed tooth germ or tooth; and
embedding the reconstructed tooth germ or tooth in the tooth-deficient area.

### BRIEF DESCRIPTION OF THE DRAWINGS

Fig. 1A shows photographic images of the outer appearance of the tooth taken at an angle of 45° with respect to the site of transplantation in the maxilla of the tooth of Example 2 of the present invention, which were taken during the eruption period (left), the period after the eruption until occlusion (center) and the period after the occlusion (right).
Fig. 1B shows photographic images of the outer appearance of the tooth taken from the direction vertical to the site of transplantation in the maxilla of the tooth of Example 2 of the present invention, which were taken during the eruption period (left), the period after the eruption until occlusion (center) and the period after the occlusion (right).
Fig. 1C shows photographic images of occlusion of the tooth of Example 2 of the present invention, which were taken during the eruption period (left), the period after the eruption until occlusion (center) and the period after the occlusion (right).
Fig. 1D shows CT images of the outer appearance of the tooth of Example 2 of the present invention, which were taken during the eruption period (left), the period after the eruption until occlusion (center) and the period after the occlusion (right).
Fig. 1E shows cross-sectional CT images (E) of the tooth of Example 2 of the present invention, which were taken during the eruption period (left), the period after the eruption until occlusion (center) and the period after the occlusion (right).
Fig. 2A is a graph showing the Knoop hardness of enamel of the erupted tooth of Example 2 of the present invention.
Fig. 2B is a graph showing the Knoop hardness of dentin of the erupted tooth of Example 2 of the present invention.
Fig. 3 is a hematoxylin-eosin stained image of the regenerated tooth of Example 2 of the present invention, which the tooth immediately before its eruption (at a magnification of ×20).
Fig. 4A is a hematoxylin-eosin stained image to confirm the bone remodeling phenomenon in the bone at the periodontal ligament (B: pressure side, C: tension side) which occurred when the orthodontic force of Example 2 of the present invention was loaded (at a magnification of ×20).
Fig. 4B is a magnified view showing the pressure side (black-framed area B) in Fig. 4A (at a magnification of ×200).
Fig. 4C is a magnified view showing the tension side (black-framed area C) in Fig. 4A (at a magnification of ×200).
Fig. 5A is an immunostained image, which shows expression of c-fos in the medulla oblongata observed in the absence of the orthodontic stimulations of Example 2 of the present invention (at a magnification of ×100).
Fig. 5B is an immunostained image, which shows expression of c-fos in the medulla oblongata observed 2 hours after the application of orthodontic stimulations of Example 2 of the present invention (at a magnification of ×100).
Fig. 5C is an immunostained image, which shows expression of c-fos in the medulla oblongata observed 48 hours after the application of orthodontic stimulations (at a magnification of ×100).
Fig. 5D is a immunostained image, which shows expression of c-fos in the medulla oblongata observed 2 hours after dental pulp exposure (at a magnification of ×100).

### BEST MODE FOR CARRYING OUT THE INVENTION

The method of the present invention of producing a restorative material is a method of producing a restorative material used to restore a tooth-deficient area in an oral cavity, the method including:
positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other;
culturing the first and second cell masses to form a reconstructed tooth germ or tooth (this step is hereinafter referred to as a "reconstructed tooth germ-formation step"); and
confirming directionality of the reconstructed tooth germ or tooth formed by the culturing so as to enable the reconstructed tooth germ or tooth to be embedded such that the tip of the tooth is faces the interior of the oral cavity (this step is hereinafter referred to as a "directionality-confirmation step");
the tooth germ or tooth whose directionality has been confirmed being used as a restorative material to obtain an equivalent of a missing tooth in the tooth-deficient area.

Further, the method of the present invention of restoring a tooth-deficient area is a method of restoring a tooth-deficient area in an oral cavity, the method including:
positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other;
culturing the first and second cell masses to form a reconstructed tooth germ or tooth (this step is hereinafter referred to as a "reconstructed tooth germ-formation step"); and
embedding the reconstructed tooth germ or tooth in the tooth-deficient area (this step is hereinafter referred to as an "embedding step").

In the method of the present invention for producing a restorative material and the method of the present invention for restoring a tooth-deficient area, the reconstructed tooth germ or tooth obtained in the reconstructed tooth germ-formation step by positioning a first cell mass and a second cell mass in a support carrier, which first and second cell masses are not mixed with each other but made to closely contact each other, is used as a restorative material to be embedded in the missing area. By embedding this reconstructed tooth germ or tooth in the missing area as a restorative material, an equivalent of a tooth erupts from the embedding area that comes to have a length and hardness that allow occlusion with the opposing tooth, and extension of nerves into the equivalent may be achieved. Therefore, the equivalent of a tooth can have hardness equivalent to a normal tooth similar to the teeth surrounding the equivalent of the tooth, an ability to achieve normal occlusion, and responsiveness to stimuli equivalent to a normal tooth.

In the present invention, the term "equivalent of a tooth" or the like is used to mean a tooth that has a length and hardness that allow occlusion with the opposing tooth when the tooth has erupted from the tooth-deficient area where the equivalent was embedded.
In the present invention, the "reconstructed tooth germ-formation step" is applied to both the method for producing a restorative material and the method for restoring a tooth-deficient area
Further, the term "step" used herein includes not only a discrete step, but also steps which cannot be clearly distinguished from another step, as long as the expected effect of the pertinent step can be achieved.
In addition, ranges indicated herein with "to" include the numerical values before and after "to".
The invention will be described below.

In the present invention, the term "tooth" means a tissue continuously having a layer of dentin inside and a layer of enamel outside, a tissue having directionality resulting from a crown and a root. The directionality of a tooth may be identified by arrangement of its crown and root. The crown and root may be visually confirmed based on their shapes, histological staining or the like. The crown means a part having a layer structure of enamel and dentin, and the enamel layer is absent in the root.

Dentin and enamel may be easily and morphologically identified by those skilled in the art by histological staining or the like. Enamel may also be identified by the presence of an ameloblast, which may be confirmed by the presence/absence of amelogenin. On the other hand, dentin may be identified by the presence of an odontoblast, which may be confirmed by the presence/absence of dentinsialoprotein. Confirmation of amelogenin and dentinsialoprotein may be carried out easily by a method well-known in the art, and examples of the method include *in situ* hybridization and immunostaining with an antibody, or the like.
Further, the directionality of a tooth may be identified based on arrangement of its crown and root. The crown and root may be visually confirmed based on their shapes, histological staining or the like.

In the present invention, the terms "tooth germ" and "tooth bud" are used to refer specifically to those distinguished based on the developmental stages. In this case, "tooth germ" means an early embryo of a tooth, which is destined to become a tooth in the future, and which is at a stage including the bud stage and the bell stage according to typical developmental staging of a tooth, and especially means such a tissue in which no accumulation of dentin and enamel is observed, which are characteristic to the hard tissue of a tooth. On the other hand, "tooth bud" means a tissue at a stage between the transitional stage from "tooth germ" used in the present invention, that is, the stage where the accumulation of dentin and enamel characteristic to the hard tissue of a tooth starts, and the stage before a tooth germinates from gingiva to exert typical functions of a tooth. Development of a tooth from a tooth germ follows each of the bud stage, the cap stage, the early bell stage and the late bell stage. Here, at the bud stage, epithelial cells invaginate such that they wrap around mesenchymal cells, and when reaching the early bell stage and the late bell stage, the epithelial cell portion becomes the outer enamel and the mesenchymal cell portion begins to form dentin internally. Therefore, a tooth is formed from a tooth germ by cell-cell interaction between epithelial cells and mesenchymal cells.

In the present invention, "mesenchymal cell" means a cell derived from a mesenchymal tissue and "epithelial cell" means a cell derived from an epithelial tissue.
Further, in the present invention, "periodontal tissue" means alveolar bone and periodontal ligament formed mainly in the outer layer of a tooth. Alveolar bone and periodontal ligament may be morphologically and easily identified by those skilled in the art by histological staining or the like.

In the reconstructed tooth germ-formation step of the present invention, a first cell mass formed from either mesenchymal cells or epithelial cells and a second cell mass formed from the other of the mesenchymal cells, one of the mesenchymal cells or epithelial cells being derived from a tooth germ, are positioned in a support carrier, the first and second cell masses being not mixed with each other but made to closely contact each other; and then the first and second cell masses are cultured to form a reconstructed tooth germ.
Regarding the reconstructed tooth germ-formation step, the method described in WO 2006/129672 can be applied as it is.

Each of the first cell mass and the second cell mass is formed from only mesenchymal cells or epithelial cells, and each of the cell masses substantially consists of one of these types of cells. The term "cell mass" means a state wherein cells are closely packed, and the cells may be either in the state of a tissue or in the state of a cell mass (cell aggregate) prepared from the state of single cells. The term "substantially consists" means that the amounts of matters included other than the cells of interest are as small as possible. The number of cells constituting each cell mass varies depending on the animal species and on the type, hardness and size of the support carrier, and may be generally 10¹ to 10⁸ cells, preferably 10³ to 10⁸ cells per cell mass.

In terms of the mesenchymal cells and the epithelial cells used in the present invention, at least one of these may be derived from a tooth germ so that they may reproduce the cell arrangement in a living body and effectively form a tooth having a specific structure and directionality, and, for secure formation of a tooth, both of the mesenchymal cells and the epithelial cells are most preferably derived from a tooth germ(s). The tooth germ is preferably at a stage from the bud stage to the cap stage in view of the fact that the cells are immature and homogeneous in terms of the stage of differentiation.

Examples of the mesenchymal cells derived from other than a tooth germ include cells derived from other mesenchymal tissues in the living body, such as, preferably, bone marrow cells not containing blood cells and mesenchymal stem cells, more preferably, mesenchymal cells in the oral cavity, bone marrow cells inside the jawbone, mesenchymal cells derived from cranial neural crest cells, mesenchymal precursor cells which can generate the mesenchymal cells, and stem cells thereof.
Examples of the epithelial cells derived from other than a tooth germ include cells derived from other epithelial tissues in the living body, such as, preferably, epithelial cells of skin, mucosa in the oral cavity, and gingiva, and more preferred examples of the epithelial cells include immature epithelial precursor cells which can produce differentiated, for example, keratinized or parakeratinized, epithelial cells such as skin, mucosa and the like. Examples of such immature epithelial precursor cells include non-keratinized epithelial cells and stem cells thereof.

The tooth germ and other tissues may be collected from the jawbone or the like of various animals, for example, primates such as humans and monkeys and ungulates such as pigs, cows and horses, which are mammals; rodents such as mice, rats and rabbits, which are small mammals; and dogs and cats and the like. For the collection of the tooth germ and the tissue, a condition generally used for collecting a tissue may be applied without modification, and the tooth germ or the tissue may be collected under sterile conditions and stored in an appropriate preservation solution. Examples of a human tooth germ include the tooth germ of a third molar, which is the so-called wisdom tooth, as well as a fetal tooth germ, and, from the viewpoint of utilization of autogenous tissues, usage of the tooth germ of a wisdom tooth is preferred. In the case of mouse, a tooth germ at an embryonic day of 10 days to 16 days is preferably used.

Preparation of mesenchymal cells and epithelial cells from this tooth germ is carried out by dividing the tooth germ isolated from its surrounding tissue, into a tooth germ mesenchymal tissue and a tooth germ epithelial tissue based on their shapes. In this process, enzymes may be used for the sake of simple isolation of the tissues. Examples of the enzymes used for such a purpose include dispase, collagenase and trypsin.

The mesenchymal cells and epithelial cells may be prepared into single cells from the mesenchymal tissue and the epithelial tissue, respectively. In order to make the cells of the tissues easily dispersible into single cells, enzymes such as dispase, collagenase and trypsin may be used.

As the medium used for the culture, a medium generally used for animal cell culture, such as Dulbecco's Modified Eagle Medium (DMEM), may be used, and serum for promotion of cell proliferation may be added, or, as an alternative to the serum, a cellular growth factor such as FGF, EGF or PDGF or a known serum component such as transferrin may be added. In cases where serum is added, its concentration may be changed appropriately depending on the culture conditions, and may usually be 10% by volume. For the cell culture, normal culture conditions, such as those for culture in an incubator at 37°C under 5% CO₂ may be applied. An antibiotic such as streptomycin may be added as appropriate.

The support carrier used in the present invention may be one in which cells can be cultured, and is preferably a mixture with the above medium. Examples of such a support carrier include collagen, agarose gel, carboxymethyl cellulose, gelatin, agar, hydrogel, elastin, fibrin, fibronectin, laminin, extracellular matrix mixtures, polyglycolic acid (PGA), polylactic acid (PLA), lactic acid/glycolic acid copolymers (PLGAs), Cellmatrix (trade name; manufactured by Nitta Gelatin Inc.), Mebiol Gel (trade name; manufactured by Ikeda Scientific Co., Ltd.) and Matrigel (trade name; manufactured by Beckton, Dickinson and Company), which may also be used in combination. These support carriers may have hardness with which the cells can be virtually maintained at the locations where they were positioned in the support carrier, and examples of the support carrier include those in the forms of a gel, fiber and solid. Among these, from the viewpoint that appropriate hardness and retentivity can be easily provided, collagen, agarose gel, carboxymethyl cellulose, gelatin, agar, hydrogel, Cellmatrix, Mebiol Gel, Matrigel, extracellular matrix mixtures, elastin, fibrin, fibronectin and laminin, and combinations thereof are more preferred; and collagen, fibrin, fibronectin, laminin, extracellular matrix mixtures, Cellmatrix, Mebiol Gel, Matrigel, and combinations thereof are still more preferred. These support carriers allow achievement of good cell-cell interactions among the mesenchymal cells and epithelial cells constituting the respective cell masses and a good interaction between the cell masses. Here, the hardness with which the cells can be maintained at their locations may be hardness which is usually applicable to three-dimensional culture, that is, hardness with which the arrangement of the cells can be maintained while hypertrophy of the cells due to their growth is not inhibited, and such hardness can be easily determined.
Here, the support carrier may have a thickness sufficient for allowing growth of the first and second cell masses inside the carrier, and the thickness may be appropriately set depending on the size of the tissue of interest, and the like.

Further, the support carrier may have a retentive capacity whereby the cells can maintain their contacting state without being dispersed. As used herein, the "contacting state" is preferably a closely-packed (high density) state which ensures the cell-cell interaction within each cell mass and between the cell masses, and such a high density state in a cell aggregate enables culturing of the cells with at a degree of retention, for example, with which a stronger contacting state than a state of simply touching can be maintained. For example, in the case of collagen, appropriate hardness is provided for usage at a final concentration of 2 to 3 mg/ml, that is, a concentration which exerts a jelly strength of 120 g to 250 g based on the method according to JIS-K6503-1996 (measured as the load necessary for depressing by 4 mm using a plunger with a diameter of 12.7 mm). The jelly strength is not limited, and other types of support carriers may also be preferably used as the support carrier of the present invention as long as these have the same strength based on the same evaluation method. Further, a support carrier having hardness corresponding to the desired jelly strength may be obtained by mixing one or more kinds of support carriers.

A high density state means a density almost equivalent to the density at which a tissue is constructed, for example, in the case of the cell masses, 5 x 10⁷ to 1 x 10⁹ cells/ml at the time of cell positioning, preferably 1 x 10⁸ to 1 x 10⁹ cells/ml to ensure the cell-cell interaction without impairing the cell activity, and most preferably 2 x 10⁸ to 8 x 10⁸ cells/ml. In order to prepare a cell mass having such a cell density, it is preferred to mass and precipitate cells by centrifugation since this conveniently enables achievement of the high density without impairing the cell activity. Such centrifugation may be carried out at a revolution speed equivalent to a centrifugal force of 300 to 1200 x g, which will not adversely affect the survival of the cells, and preferably 500 to 1000 x g, for 3 to 10 minutes. Centrifugation at lower than 300 x g may lead to insufficient precipitation of the cells and the cell density may become low, while centrifugation at higher than 1200 x g may cause damage to the cells, and therefore both of these cases are not preferred.

In cases where high density cells are prepared by centrifugation, the centrifugation is normally carried out after preparing a suspension of single cells in a container such as a tube used for cell centrifugation, and the supernatant is removed to the greatest extent possible, leaving the cells as the precipitates.
In cases where the precipitates are prepared by centrifugation, these may be directly positioned inside the support carrier. Here, the volume of components other than the cells of interest (for example, a culture medium, a buffer solution, the support carrier or the like) is preferably not more than the volume of the cells, and most preferably, components other than the cells of interest are not contained. In such a high density cell mass, cells are in close contact with each other and the cell-cell interaction may be effectively exerted. Especially, in cases where a cell mass containing only an extremely small amount of components other than the cells of interest is positioned inside the support carrier, the cells further aggregate due to solidification of the support carrier and the like, to provide a state wherein the cells are more tightly packed.

The closer the contact between the first cell mass and the second cell mass, the better, and it is especially preferred that the second cell mass be positioned such that it presses against the first cell mass. Further, wrapping around the first cell mass and the second cell mass with a solid which does not inhibit a culture medium or oxygen permeation is also effective in making the contact between the cell masses closer. It is also preferred to add a high-density cell suspension to a solution having a different viscosity to position the cell suspension therein, followed by solidification of the solution without any change, since this may conveniently achieve maintenance of contacting of the cell. Here, in cases where the first cell mass is an mass of single tooth germ mesenchymal cells and the second cell mass is a tooth germ epithelial tissue, it is preferred to position the enamel knot of the tooth germ epithelial tissue in contact with the first cell mass, but the present invention is not limited to this.

When the support carrier is in the form of a gel, solution or the like, the positioning of the cells in the support carrier may be followed by a solidification step in which the support carrier is solidified. For solidification of the support carrier, conditions generally used for solidification of the support carrier may be applied without modification. For example, in cases where a solidifiable compound such as collagen is used for the support carrier, solidification can be achieved under conditions generally applied, for example, by being left to stand at the culture temperature for several minutes to several tens of minutes. By this, adhesions between the cells inside the support carrier can be fixed and made robust.

The time period of the culture for formation of a reconstructed tooth germ varies depending on the number of cells positioned arranged in the support carrier and the states of the cell masses, the conditions under which the culture is carried out, as well as on the species of the animal, and may be generally preferably at least 1 day, more preferably not less than 3 days. A reconstructed tooth germ or tooth obtained by culture for this time period can erupt as a functional tooth even after being embedded in the missing area.
Further, by extending this culture period, the process of formation of a reconstructed tooth germ, that is, accumulation of dentin and enamel, formation of a crown and formation of a root, proceeds. Therefore, this culture period for formation of a reconstructed tooth germ can be appropriately controlled depending on the conditions under which the culture is carried out, the animal species, the condition of the reconstructed tooth germ, and the like. For example, when the organ culture described below is carried out, one may proceed to the embedding step after 7 days, or, in some cases, 6 days, of the culture. Alternatively, depending on the condition of the reconstructed tooth germ, or when another culture method is used, the culture period may be longer than 30 days, or, in some cases, longer than 50 days, longer than 100 days, or longer than 300 days.

The culture in the support carrier may be carried out either only with the support carrier containing the first and second cell masses therein, or in the presence of other animal cells.
When the culture in the support carrier is carried out only with the support carrier, it may be carried out under conditions generally used for culturing of animal cells. For such a culture, in general, the conditions for culturing of animal cells may be applied without modification, and the above-mentioned conditions can be applied without modification. Further, serum derived from mammals, and various cellular factors which are known to be effective in growth and differentiation of these cells may be added to the culture. Examples of such cellular factors include FGF and BMP.

Further, from the viewpoint of gas exchange and nutrient supply for tissues and cell masses, and from the viewpoint that the preparation can be carried out in vitro throughout the whole process without contacting of the cells with other animal cells, the culture in the support carrier is preferably carried out as organ culture. In organ culture, generally, culturing is performed by floating porous membrane on a culture medium suitable for growth of animal cells and placing the first and second cell masses embedded in a support carrier on the membrane. The porous membrane used herein is preferably a membrane having many pores with a diameter of 0.3 to 5 µm, and specific examples thereof include Cell Culture Insert (trade name) and Isopore Filter (trade name).
The culture period in organ culture may be about 1 day to 7 days, and may be preferably 3 to 6 days.

When the culture in the support carrier is carried out in the presence of other animal cells, a tooth having a specific cell arrangement can be formed at an early stage in response to the actions of various cytokines and the like from the animal cells. Such culture in the presence of other animal cells may be performed by culture ex vivo using isolated cells or cultured cells.

Preferred examples of animals which can be used for this application include mammals such as humans, pigs and mice, and the species is preferably the same as that from which the tooth germ tissue was obtained, or another species which was altered to be immunodeficient. When a living body is used, preferred examples of transplantation to a suitable site in the living body include subrenal capsule, mesenteric and subcutaneous transplantation, which allow an organ or tissue composed of animal cells to develop as normally as possible. The period of the culture in such presence of animal cells may be appropriately controlled depending on the origin of the cells, conditions of the culture, the animal species to which the transplantation is carried out, and the like, as in the case of the organ culture described above.

By such a reconstructed tooth germ-formation step, a tooth germ which may have a cell arrangement (structure) specific to a tooth, that is, dentin inside and enamel outside, or a tooth having these, can be obtained. Further, the tooth germ or the tooth also preferably has directionality, that is, a tip (crown) and a root of a tooth.
Both the first cell mass and the second cell mass are preferably masses of single cells since an aggregate of teeth composed of plural teeth having a cell arrangement specific to a tooth can be obtained. When such an aggregate of teeth was obtained, each tooth is separated from the aggregate before its use.

In the directionality-confirmation step in the method of production of a restorative material of the present invention, the directionality of the tooth germ or tooth obtained in the reconstructed tooth germ-formation step described above is confirmed so as to enable the tooth germ or tooth to be embedded in the tooth-deficient area such that the tip of the tooth faces the interior of the oral cavity.

The directionality of the tooth germ or tooth means the directionality with which the tooth germ or tooth is embedded in the tooth-deficient area. When formation of a crown is found, the crown is confirmed to be facing the interior of the oral cavity, and when formation of a crown cannot be found, the epithelial cell layer in the portion corresponding to the crown or the epithelial cell layer in the reconstructed tooth germ is confirmed to be facing the interior of the oral cavity. Alternatively, the open portion of the epithelial/mesenchymal cell layer of the reconstructed tooth germ may be confirmed to be at the opposite side with respect to the oral cavity. By positioning the tooth germ or tooth in the tooth-deficient area according to the directionality confirmed as above, the tip (crown) of the tooth can be made to face the interior of the oral cavity, so that the direction of the tooth becomes the same as that of the surrounding teeth.

The tooth germ or tooth whose directionality has been confirmed in this directionality-confirmation step is used as a restorative material to obtain an equivalent of the missing tooth in the tooth-deficient area.
That is, the restorative material containing the tooth germ or tooth after confirmation of its directionality is embedded in the tooth-deficient area. The tooth germ or tooth in the restorative material grows thereafter in the missing area over time and erupts as an equivalent of a tooth. The tip (cusp) of the erupted tooth equivalent reaches almost the same position as those of the surrounding teeth (occlusal plane), and the tooth equivalent does not extend further thereafter, although the situation varies depending on the state, size and the like of the tooth germ or tooth in the restorative material. The length of the period during which the tooth germ or tooth remains embedded until eruption and occlusion is different among animal species and varies depending on the condition of the embedded tooth germ or tooth.

The erupted tooth equivalent has hardness similar to that of a native tooth. The hardness is an index that indicates the extent to which the dentin and the enamel of the tooth equivalent tolerate deformation due to compression, abrasion and/or the like, and can be confirmed by measurement of the Knoop hardness. The Knoop hardness of enamel of a normal tooth in an adult mouse is 300 to 600 KHN, preferably 300 to 500 KHN, and the Knoop hardness of normal dentin is 60 to 120 KHN. The tooth equivalent obtained in the present invention has Knoop hardness within these ranges. By this, masticatory function equivalent to that of a normal tooth is exerted.

Further, the erupted tooth equivalent has dental pulp and periodontal ligament in addition to enamel and dentin, showing a tissue constitution similar to that of a normal tooth. Since the tooth has the periodontal ligament, nerves extend, so that responsiveness to noxious stimuli such as compression to the tooth is maintained. Such a tissue constitution can be confirmed visually or by histological analysis, immunostaining, analysis of gene expression, or the like according to a conventional method.

Thus, the restorative material obtained by the method of the present invention for producing a restorative material can be used as a raw material for a tooth equivalent having a length and hardness which allow its occlusion with the opposing tooth.

In the embedding step in the method of the present invention for restoring a tooth-deficient area, a tooth germ or tooth obtained by the reconstructed tooth germ-formation step described above is embedded in a tooth-deficient area as a restorative material.
Here, in terms of the direction in which the tooth germ or tooth is embedded, the tooth germ or tooth is preferably positioned in the same manner as in the directionality-confirmation step in the above-mentioned method for production of a restorative material, such that the crown faces the interior of the oral cavity when formation of a crown is found, or such that the epithelial cell layer in the portion corresponding to the crown or the epithelial cell layer in the reconstructed tooth germ faces the interior of the oral cavity when formation of a crown cannot be found, or alternatively, such that the open portion of the epithelial/mesenchymal cell layer of the reconstructed tooth germ is positioned at the opposite side with respect to the oral cavity. By this, the tip (crown) of the tooth equivalent can be made to face the interior of the oral cavity, so that the direction of the tooth equivalent becomes the same as that of the surrounding teeth.

The size and the depth of the missing area are usually not restricted as long as the area is formed in gingiva caused by extraction of a tooth, or the like, and the shape of the missing area is not restricted. As long as a regenerated tooth germ can be embedded in the area, these can be appropriately controlled depending on the missing place, subject animal species, type of the tooth of interest, and the like.
Such a missing area is usually positioned on the jawbone, alveolar bone of the oral cavity, or the like. When the mass of the alveolar bone is decreased due to loss of a tooth, the bone mass may be increased by bone regeneration according to a known method clinically used for placement of an implant, such as the GTR (guided tissue regeneration) method. The positioning of the tooth germ or tooth in the hole portion is preferably followed by suture or the like according to a conventional process.

Thereafter, the embedded tooth germ grows with time to become a tooth in the missing area, followed by its eruption as a tooth equivalent. Similarly to the matters described above for the method for production of a restorative material, the tip (cusp) of the erupted tooth equivalent reaches almost the same position as those of the surrounding teeth (occlusal plane), and the tooth equivalent does not extend further thereafter, although the situation varies depending on the state, size and the like of the tooth germ to be embedded. By this, the length of the tooth can be made to allow occlusion of the tooth with the opposing tooth. The length of the period during which the tooth germ or tooth remains embedded until eruption and occlusion is different among animal species and varies depending on the condition of the embedded tooth germ or tooth.

Similarly to the fact described above for the method for production of a restorative material, the erupted tooth equivalent has hardness similar to that of a native tooth. The hardness is an index that indicates the extent to which the dentin and the enamel of the tooth equivalent tolerate deformation due to compression, abrasion and/or the like, and can be confirmed by measurement of the Knoop hardness. The Knoop hardness of enamel of a normal tooth in an adult mouse is 300 to 600 KHN, preferably 300 to 500 KHN, and the Knoop hardness of normal dentin is 60 to 120 KHN. The tooth equivalent obtained in the present invention has Knoop hardness within these ranges. By this, masticatory function equivalent to that of a normal tooth is exerted.

Further, similarly to the fact described above for the method for production of a restorative material, the erupted tooth equivalent has dental pulp and periodontal ligament in addition to enamel and dentin, showing a tissue constitution similar to that of a normal tooth. Since the tooth has the periodontal ligament, nerves extend, so that responsiveness to noxious stimuli such as compression to the tooth is maintained. Such a tissue constitution can be confirmed visually or by histological analysis, immunostaining, analysis of gene expression, or the like according to a conventional method.

In the method of the present invention for restoring an area missing a tooth, a tooth-deficient area is restored using a tooth germ or tooth reconstructed as described above, and therefore the tooth-deficient area can be restored such that normal occlusion, and responsiveness to noxious stimuli equivalent to that of a normal tooth, are attained. As a result, the tooth equivalent regenerated in the missing area acts as a functional tooth, and the restored state can be maintained for a long period of time.
This enables maintenance of quality of life, and therefore the present method is suitably used in the field of esthetics. Further, since deterioration of health conditions due to the existence of a tooth-deficient area in the oral cavity can be avoided, the health conditions of non-human mammals such as livestock including cows, horses and pigs and pet animals including dogs and cats can be maintained.

### EXAMPLES

Examples of the present invention are now described, but the present invention is not limited thereto. "%" in Examples is by weight (mass) unless otherwise specified.

### Example 1

### (1) Preparation of Tooth Germ Epithelial Cells and Tooth Germ Mesenchymal Cells

In order to form a tooth, a tooth germ was reconstructed. Mouse was used as a model of this experiment.
A tooth germ tissue of a mandibular molar was isolated from an embryo of a C57BL/6N mouse (purchased from CLEA Japan, Inc.) at the embryonic day of 14.5 days under the microscope according to a conventional method. The tooth germ tissue of a mandibular molar was washed with Ca²⁺/Mg²⁺-free phosphate buffer (PBS(-)), and treated at room temperature for 12.5 minutes with an enzyme solution prepared by adding Dispase II (Roche, Mannheim, Germany) to PBS(-) to a final concentration of 1.2 U/ml. The tooth germ tissue was then washed 3 times with DMEM (Sigma, St. Louis, MO) supplemented with 10% FCS (JRH Biosciences, Lenexa, KS). Further, a DNase I solution (Takara, Siga, Japan) was added to a final concentration of 70 U/ml to disperse the tooth germ tissue, and the tooth germ epithelial tissue and the tooth germ mesenchymal tissue from each other were separated using a 25G injection needle (Terumo, Tokyo, Japan).

In terms of tooth germ epithelial cells, the tooth germ epithelial tissue obtained as described above was washed 3 times with PBS(-), and subjected to 2 times of treatment at 37°C for 20 minutes with an enzyme solution which Collagenase I (Worthington, Lakewood, NJ) was dissolved at a final concentration of 100 U/ml in PBS(-). The cells were collected by precipitation by centrifugation, and treated with 0.25% Trypsin (Sigma)-PBS (-) at 37°C for 5 minutes. The cells were washed 3 times with DMEM supplemented with 10% FCS, and a DNase I solution was added to the cells to a final concentration of 70 U/ml, followed by pipetting to obtain single tooth germ epithelial cells.
On the other hand, in terms of tooth germ mesenchymal cells, the tooth germ mesenchymal tissue was washed 3 times with PBS(-), and subjected to treatment with PBS(-) containing 0.25% Trypsin (Sigma) and 50 U/ml Collagenase I (Worthington). DNase I (Takara) was added to the resultant to 70 U/ml, followed by pipetting to obtain single tooth germ mesenchymal cells.

(2) Preparation of Reconstructed Tooth Germ
Subsequently, using the tooth germ epithelial cells and the tooth germ mesenchymal cells prepared as described above, a tooth germ was reconstructed. In a 1.5 mL microtube (Eppendorf, Hamburg, Germany) to which silicone grease was applied, the tooth germ epithelial cells or the tooth germ mesenchymal cells suspended in DMEM (Sigma) supplemented with 10% FCS (JRH) were placed, and the cells were recovered by centrifugation as a precipitate. The supernatant of the culture medium after the centrifugation was removed as much as possible, and centrifugation was carried out again, followed by complete removal of the culture medium remaining around the precipitate of the cells under a stereoscopic microscope using GELoader Tip 0.5-20 µL (Eppendorf). Thereafter, the cells were dispersed by tapping or pipetting, to prepare cells to be used for preparation of a reconstructed tooth germ.

To a petri dish to which silicone grease was applied, 30 µL of Cellmatrix type I-A (Nitta gelatin, Osaka, Japan) was added dropwise to prepare a collagen gel droplet. To this solution, 0.2 to 0.3 µL of the above tooth germ epithelial cells or tooth germ mesenchymal cells were applied using a 0.1-10 µL pipette tip (Quality Scientific plastics) to prepare a cell aggregate (5×10⁴ cells/aggregate). In terms of a reconstructed tooth germ, tooth germ epithelial cells were applied in the same manner such that these cells were brought into contact with the cell aggregate of tooth germ mesenchymal cells prepared above, to prepare a cell aggregate, thereby preparing a reconstructed tooth germ wherein the both are in close contact with each other.

The reconstructed tooth germ prepared in the gel was left to stand in a CO₂ incubator for 10 minutes to solidify Cellmatrix type I-A (Nitta Gelatin). A culture vessel was prepared such that DMEM (Sigma) supplemented with 10% FCS (JRH) was in contact with Cell Culture Inserts (PET membrane having a pore size of 0.4 µm;
BD, Franklin Lakes, NJ). Onto the membrane of Cell Culture Inserts in the culture vessel, the cell aggregate was transferred together with the surrounding gel as a support carrier, followed by organ culture for 18 to 24 hours. After the culture, organ culture was continued in the Cell Culture Inserts to analyze development of a tooth.

(3) Method of Individual Separation
On Day 2 to 5 during the organ culture, a reconstructed tooth germ in which plural tooth germs were developing was subjected to surgical separation into individual tooth germs under a stereoscopic microscope using an injection needle and forceps. To a petri dish to which silicone grease was applied, 30 µL of Cellmatrix type I-A (Nitta gelatin, Osaka, Japan) was added dropwise to prepare a collagen gel droplet. Each individually separated tooth germ was placed in this solution, and left to stand in a CO₂ incubator for 10 minutes to solidify Cellmatrix type I-A (Nitta Gelatin). A culture vessel was prepared such that DMEM (Sigma) supplemented with 10% FCS (JRH) was in contact with Cell Culture Inserts (PET membrane having a pore size of 0.4 µm; BD, Franklin Lakes, NJ). Onto the membrane of Cell Culture Inserts in the culture vessel, the individually separated tooth germ was transferred together with the surrounding gel as a support carrier, followed by organ culture for 18 to 24 hours.

(4) Intraoral Transplantation
From the individually separated tooth germ prepared as described above, the surrounding gel was surgically removed using an injection needle and forceps. The individually separated tooth germ was transplanted to alveolar bone in the first molar (M1) region of the upper jaw of C57BL/6 of 7 to 10 weeks old, without being transplanted to subrenal capsule. By the present invention, an individual tooth was allowed by erupt in the oral cavity, to realize oral function such as mastication.

### Example 2

### (1) Preparation of Individually Separated Tooth Germ

Organ culture was carried out in the same manner as in Example 1 (1) to (2), and on Day 2 to 5 during the organ culture, a reconstructed tooth germ in which plural tooth germs were developing was subjected to surgical separation into individual tooth germs under a stereoscopic microscope using an injection needle and forceps. In terms of the individually separated tooth germ, to a petri dish to which silicone grease was applied, 30 µL of Cellmatrix type I-A (Nitta gelatin, Osaka, Japan) was added dropwise to prepare a collagen gel droplet. Each individually separated tooth germ was placed in this solution, and left to stand in a CO₂ incubator for 10 minutes to solidify Cellmatrix type I-A (Nitta Gelatin). A culture vessel was prepared such that DMEM (Sigma) supplemented with 10% FCS (JRH) was in contact with Cell Culture Inserts (PET membrane having a pore size of 0.4 µm; BD, Franklin Lakes, NJ). Onto the membrane of Cell Culture Inserts in the culture vessel, the individually separated tooth germ was transferred together with the surrounding gel as a support carrier, followed by organ culture for 5 days. When plural reconstructed tooth germs were formed, these were surgically separated into single reconstructed tooth germs. After the culture, the surrounding gel was surgically removed using an injection needle and forceps, and the tooth germ was transplanted to alveolar bone in the first molar (M1) region of the upper jaw of C57BL/6 of 8 weeks old.

(2) Method of Intraoral Transplantation
Three days before intraoral transplantation, 240 µl/20 g body weight of physiological saline containing 5 mg/ml sodium pentobarbital was intraperitoneally injected to an 8-week old C57BL/6 which had been subjected to inhalation anesthesia with diethyl ether. An upper jaw M1 of the mouse under anesthesia was extracted from the upper jaw using forceps, and absence of a residual root was confirmed, followed by wiping spouting blood with absorbent cotton to stop bleeding. In terms of food ingestion, the mouse was fed with powdered diet every day. The mouse was kept for not less than 3 weeks to allow curing of the extraction wound, to prepare C57BL/6 lacking M1.

The C57BL/6 lacking M1, prepared by the method described above, was subjected to inhalation anesthesia with diethyl ether, and 240 µl/20 g body weight of physiological saline containing 5 mg/ml sodium pentobarbital was intraperitoneally injected to the mouse. The mouse under anesthesia was fixed on its back on a dissecting table, and the upper and lower jaws were fixed using rubbers or threads such that the mouth was fully open. The gingiva at the alveolar bone crest in the upper jaw M1 region was incised using a scalpel, and peeled off together with periosteum to expose the mandible. Using a pin vise (extra fine drill), a hole having a diameter of 1 mm was prepared in the region of the mandible corresponding to M 1 such that perforation of the maxillary sinus was not caused, and the individually separated tooth germ was transplanted to the hole. After the transplantation, the wound was closed with the periosteum/gingival flap, and sutured with thread with an attached needle. In order to control the direction of the individually separated tooth germ to be transplanted, the cusp of the individually separated tooth germ was labeled with methylene blue, and the transplantation was carried out such that the label was facing the direction of eruption. The 8-week old C57BL/6 subjected to the intraoral transplantation was fed with powdered diet every day

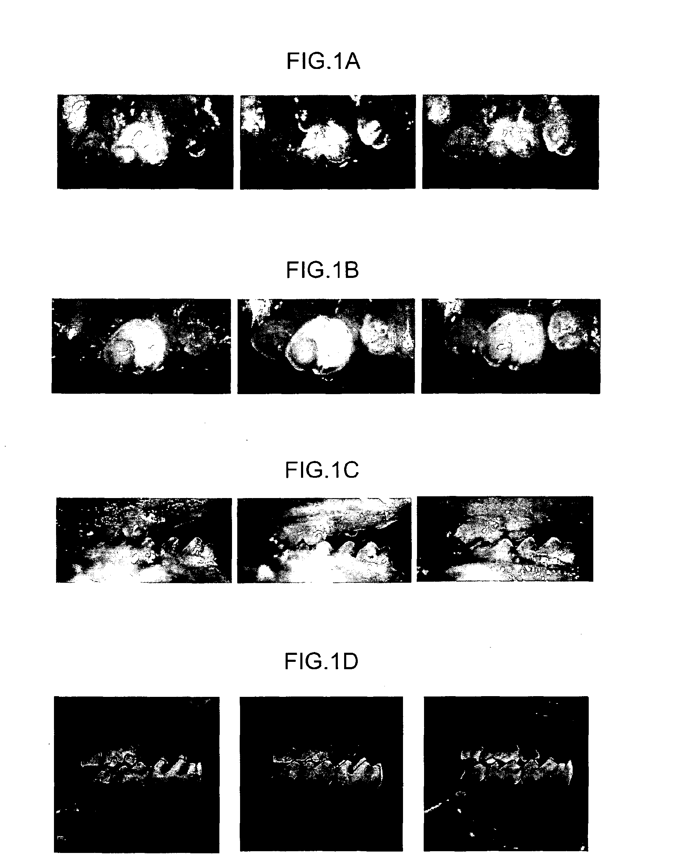

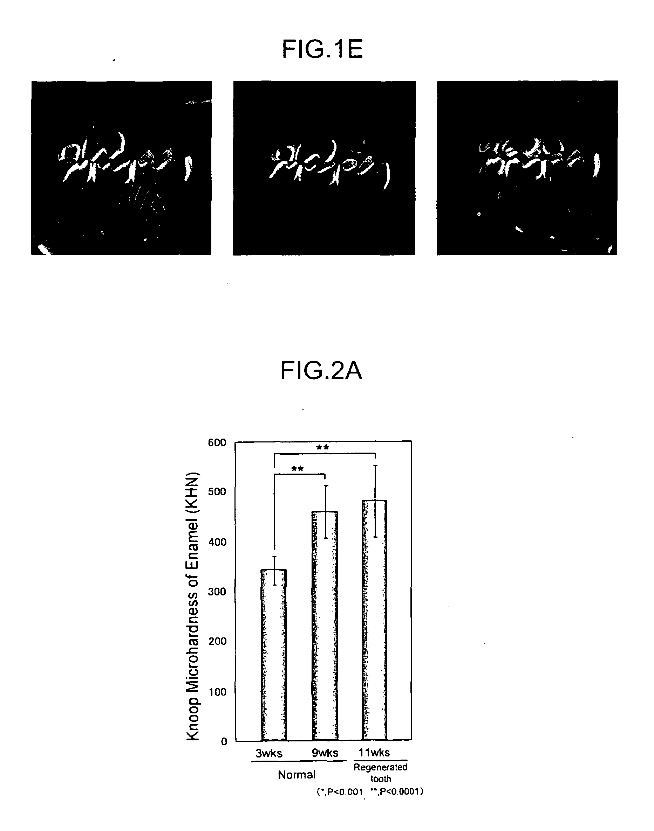

To the C57BL/6 to which an individual tooth germ was transplanted by the method described above, 240 µl/20 g body weight of physiological saline containing 5 mg/ml sodium pentobarbital was intraperitoneally injected. The mouse under anesthesia was fixed on its back on a dissecting table, and the upper and lower jaws were fixed using rubbers such that the mouth was fully open. The regenerated tooth, the distal areas of M2 and M3, and the surrounding gingiva were observed before eruption, immediately before eruption, immediately after eruption, during eruption, and when the tooth reached the occlusal surface. The observation results are shown in Fig. 1. In each of Fig. 1A to Fig. 1E, the left panel shows the result obtained during the eruption period (before eruption to immediately after eruption); the middle panel shows the result obtained during the period after eruption to occlusion; and the right panel shows the result obtained during the period after occlusion. In each panel of each of Fig. 1A to Fig. 1E, the regenerated tooth is shown on the right side of the panel.

About 20 to 50 days after the transplantation, the cusp of the regenerated tooth could be observed at the M1 gingiva region, indicating that eruption has started. The regenerated tooth increased its crown height over time and reached the occlusal plane. The regenerated tooth which reached the occlusal plane attained intercuspation with the opposing tooth (see Fig. 1A to Fig. 1C).
From these results, it was revealed that the regenerated tooth forms a stable occluding relation similarly to a normal tooth and has a masticatory function.

(3) MicroCT Analysis
The head containing the maxilla to which the individually separated tooth germ was transplanted was removed, and the whole head was fixed in 4% paraformaldehyde-phosphate buffer for 12 hours, followed by CT imaging using inspeXio SMX-90CT (manufactured by Shimadzu Corporation). The CT imaging was carried out with the settings of: view number, 600; average, 10; scan, 1; image, 512×512; scaling, 50; slice thickness, 1; and without BHC. The data obtained by the CT imaging were 3D-reconstructed using an analysis software Imaris (manufactured by Zeiss), to prepare cross-sectional images of MicroCT.

As a result of the MicroCT analysis, it was revealed, as shown in Fig. 1D and Fig. 1E, that, in the eruption process, the regenerated tooth longitudinally moves while forming its root, and erupts from the alveolar bone crest, after which the tooth reaches the occlusal plane. A void corresponding to the periodontal ligament could be observed between the root portion and the alveolar bone, and, in the cross-sectional view of the tooth, the pulp cavity existed and an apical foramen opens in the root apex, similar to a normal tooth. It was revealed that the regenerated tooth attained intercuspation with the opposing tooth in the occluded state. From these results, it was revealed that, during the eruption process, the regenerated tooth longitudinally moves in the alveolar bone with retaining the periodontal ligament and the pulp cavity, and reaches the occlusal plane, similarly to a normal tooth.

Further, similarly, M3 in the upper and lower jaws in the head fixed in 4% paraformaldehyde-phosphate buffer were exposed, and intraoral images at the centric occlusion position was taken according to a conventional method wherein the horizontal angle was determined based on the plane between the occlusal surfaces of the mandibular M3 and the mandibular M1, and the vertical angle was determined based on the overlap between the mesial buccal cusp and the mesial lingual cusp of the mandibular M 1 seen from the lateral direction.

As a result, it was confirmed that the regenerated tooth was occluding with the distal cusp of the mandibular M1, establishing a flat occlusal plane together with the maxillary M2 and M3. From these results, it was suggested that the erupted regenerated tooth has an established occlusal plane which is appropriate for induction of the mandibular position.

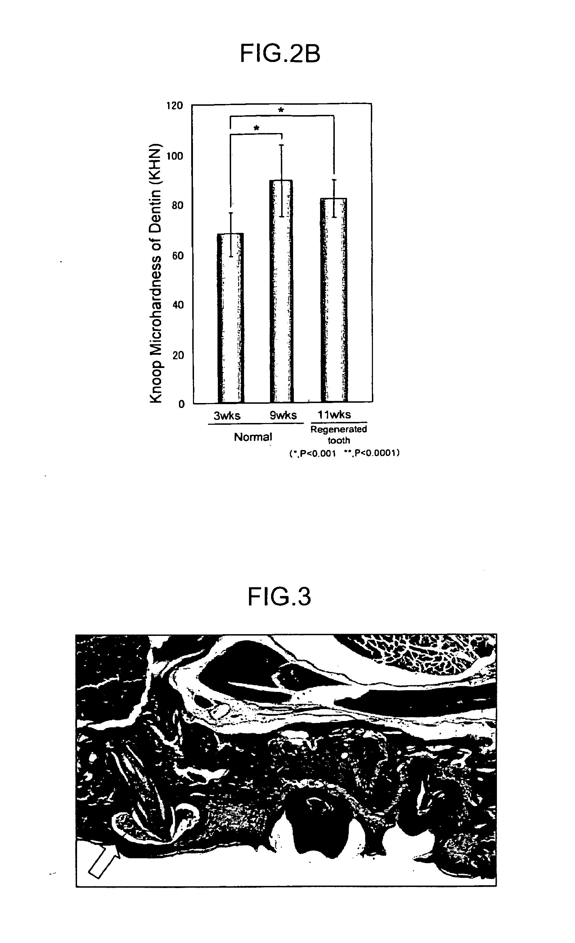

(4) Knoop Hardness
An individual tooth which was allowed to develop in the oral cavity in Example 2(1) was recovered, and the hardness of each of enamel and dentin of the tooth was measured for the identical tooth at not less than 3 positions.
The measurement was carried out using a microhardness tester (HM-102, manufactured by Mitutoyo Corporation) equipped with a quadrangular pyramidal diamond indenter for measurement of Knoop Hardness having two vertically opposite angles 172°30' and 130° (19BAA061, manufactured by Mitutoyo Corporation). The indenter was pressed into the regenerated tooth with a load of 10 g for 10 seconds, and the hardness was calculated from the length of the longer side of the 7.11:1 rhombic impression. The regenerated tooth was fixed on a metal plate using a dental resin (Unifast III, manufactured by GC CORPORATION). Measurement for enamel was carried out for a portion parallel to the ground, and measurement for dentin was carried out by cutting the tooth in the direction horizontal to the ground using a dental mechanical engine (ULTIMATE 500, manufactured by NAKANISHI INC.) to expose the surface to be tested. The results of the measurement of the Knoop hardness are shown in Fig. 2.

As shown in Fig. 2, the average Knoop hardness of enamel of a normal tooth was 341.083 KHN at 3 weeks old, 457.5 KHN at 6 weeks old, and 436 KHN at 9 weeks old; and the average Knoop hardness of enamel of the regenerated tooth was 469.81 KHN (see Fig. 2A). The average Knoop hardness of dentin of a normal tooth was 66.87 KHN at 3 weeks old, 76.53 KHN at 6 weeks old, and 88.58 KHN at 9 weeks old; and the average Knoop hardness of dentin of the regenerated tooth was 81.83 KHN (see Fig. 2B). Thus, the regenerated tooth was suggested to have hardness suitable for exerting normal masticatory function.

(5) Histological Analysis
About 20 to 50 days after the transplantation, the cusp of the regenerated tooth could be observed at the M 1 gingiva region, indicating that eruption has started. The state of eruption was observed, and the maxilla before eruption, the maxilla immediately before eruption, the maxilla immediately after eruption, the maxilla during eruption, and the maxilla when the tooth reached the occlusal surface were removed. The maxillae were fixed in 4% paraformaldehyde-phosphate buffer for 16 hours and decalcified in 22.5% formic acid for 72 hours, followed by paraffin embedding according to a conventional method and preparing 10-µm sections. The amount of the decalcifying fluid was 50 ml per two maxillae, and the total volume was replaced at 48 hours during the decalcification. Histological analysis was carried out by hematoxylin-eosin staining according to a conventional method.

Fig. 3 shows the regenerated tooth immediately before eruption (Fig. 3, white arrow). As shown in Fig. 3, a tissue structure equivalent to that of a normal tooth, having enamel, dentin, the dental pulp and the periodontal ligament could be observed. In terms of the respective tissues, the enamel had enamel prisms radially extending side by side, and the dentin was found to have dentinal tubules. The periodontal ligament had a sufficient thickness, and had a structure with which the masticatory force can be buffered. During the eruption process, it was observed that the regenerated tooth longitudinally moved while forming its root, and erupted from the alveolar bone crest, subsequently reaching the occlusal plane. During this process, the periodontal ligament was retained, suggesting that the regenerated tooth is in harmony with the periodontal tissue.

(6) Analysis of Remodeling of Bone by Orthodontic Force
Since a tooth is bound to the surrounding alveolar bone via the periodontal ligament, application of orthodontic force to a tooth causes transmission of the mechanical stress to the surrounding environment via the periodontal ligament. In the area of the periodontal ligament compressed by the orthodontic force, absorption of the alveolar bone by osteoclasts occurs, and, in the area of the periodontal ligament which has undergone traction, osteogenesis by osteoblasts occurs, thereby keeping the spatial distance between the tooth and the alveolar bone constant. For the purpose of evaluation of such functions of the periodontal ligament, orthodontic force was applied to the regenerated tooth and remodeling of the bone, such as emergence of osteoclasts in the compressed side and emergence of osteoblasts in the side which has undergone traction, caused by movement of a normal tooth, were confirmed by the following analysis.

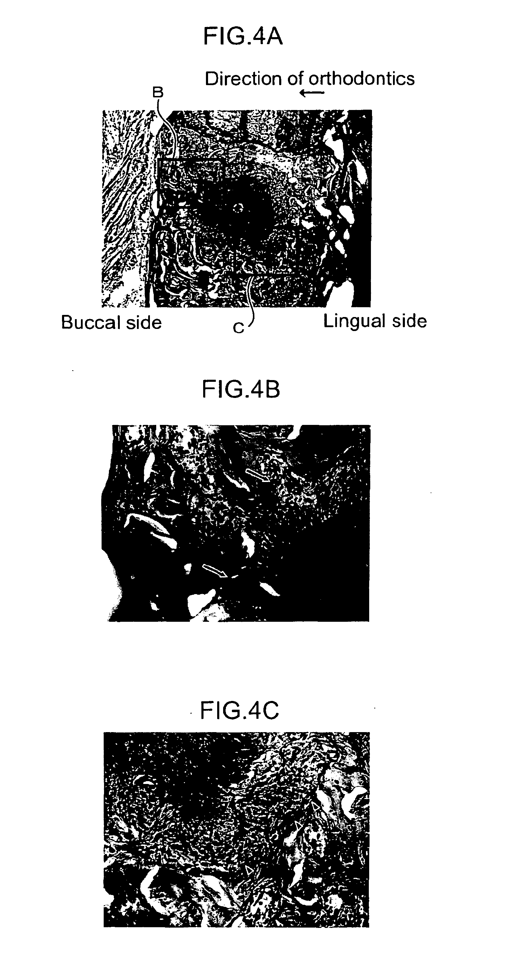

Using nickel-titanium wires having a diameter of 0.012 inch (VIM-NT orthodontic wire, round type, Oral Care), orthodontic wires for buccal movement were formed such that one end of each wire was bent to form a loop which can be adapted and fitted to the neck of either incisor in the maxilla. To C57BL/6 to which an individual tooth germ was transplanted by the method described above, 240 µl/20 g body weight of physiological saline containing 5 mg/ml sodium pentobarbital was intraperitoneally injected. The mouse under anesthesia was fixed on its back on a dissecting table, and the upper and lower jaws were fixed using rubbers or threads such that the mouth was fully open. The length of the end of the wire opposite to the loop was adjusted such that the end reaches the distal area of the erupted tooth. The loop of the orthodontic wire for buccal movement was fitted to the neck of either incisor in the maxilla, while adapting the other end of the wire to the buccal-side neck of the tooth to be subjected to orthodontics, which loop was then bonded and fixed with UNIFIL FLOW (photopolymerizable resin, manufactured by GC CORPORATION). The end of the wire which had been adapted to the buccal-side neck of the tooth was moved to the lingual-side neck of the tooth to activate orthodontic force. Subsequently, histological analysis to analyze the bone remodeling phenomenon caused by the periodontal ligament was carried out on Day 6 after the orthodontics when the bone remodeling phenomenon is especially remarkable. The results are shown in Fig. 4A to Fig. 4C.

To analyze the responsive function of the periodontal ligament of the regenerated tooth against mechanical stress, orthodontic force to cause movement to the buccal side was applied to the regenerated tooth, and the tissue image of remodeling of the bone was observed with hematoxylin-eosin-stained images.
As a result, in the buccal side, compression of the periodontal ligament, wherein periodontal ligament fibers are compressed due to narrowing of the periodontal space, was observed (see the black-framed area B in Fig. 4A, and Fig. 4B). On the other hand, in the opposite side, that is, the lingual side, the periodontal space expanded, causing traction of the periodontal ligament, wherein periodontal ligament fibers became tense (see the black-framed area C in Fig. 4A, and Fig. 4C). Further, since a single layer of cells aligned along the alveolar bone wall in the tension side of the periodontal ligament was observed, emergence of osteoblasts which form the bone (see arrowheads in Fig. 4C) was suggested. Further, in the pressure side, which is opposite to the tension side, multinucleated giant cells having a number of nuclei in the cell body were observed in the alveolar bone, and cavities formed by bone resorption were observed, so that expression of osteoclasts (see arrows in Fig. 4B) and bone resorption were suggested.
From these results, it was suggested that, in the regenerated tooth, mechanical stress such as orthodontic force causes response of the periodontal ligament, inducing the bone remodeling phenomenon, as in a normal tooth.

(8) Analysis of Pain Stimulation
Pain due to compression of teeth is well-known to be caused by orthodontics and the like, and it has been revealed that the pain is mediated by nerves existing especially in the periodontal ligament, and that production of the c-Fos protein increases in nerve cells in trigeminal spinal subnucleus caudalis (the medullary dorsal horn) (Byers MR. et al., Crit Rev Oral Biol Med 10, 4-39, 1999; Deguchi T. et al. J Dent Res 82:677-681, 2003; Fujiyoshi, Y. et al., Neuroscience Letters 283, 205-208, 2000). Further, pain due to exposure of dental pulp is well-known to be caused by the dental pulp exposure treatment, and it has been revealed, as in the case of the pain due to orthodontics, that the pain is mediated by nerves existing especially in dental pulp, and that production of the c-Fos protein increases in nerve cells in trigeminal spinal subnucleus caudalis (the medullary dorsal horn) (Byers MR. et al., Crit Rev Oral Biol Med 10, 4-39, 1999; Deguchi T. et al. J Dent Res 82:677-681, 2003). Thus, in order to analyze nerve function of the regenerated tooth, the regenerated tooth of the mouse was subjected to compressive stimulation similar to that applied during orthodontics, and the dental pulp exposure treatment by drilling a hole in dentin with a dental drill, and whether or not expression of the c-Fos protein increases in trigeminal spinal subnucleus caudalis (the medullary dorsal horn) was analyzed.

To C57BL/6 to which an individual tooth germ was transplanted by the method described above, 200 µl/20 g body weight of physiological saline containing 5 mg/ml sodium pentobarbital was intraperitoneally injected. The mouse under anesthesia was fixed on its back on a dissecting table.
Two hours and 48 hours after the activation of orthodontic force by the above method, and 2 hours after the dental pulp exposure treatment, the C57BL/6 was subjected to perfusion fixation in 4% paraformaldehyde-phosphate buffer. The isolated medulla oblongata was fixed in 4% paraformaldehyde-phosphate buffer for 16 hours, and embedded in OCT compound (Miles Inc., Naperville, IL) according to a conventional method, followed by preparing 50-µm sections using a cryostat (Leica, Wetzlar, Germany). The prepared sections were subjected to immunostaining using c-fos (SANTA CRUZ BIOTECHNOLOGY, INC., Santa Cruz, California, USA) as a primary antibody, Goat IgG fraction to rabbit IgG (CAPPEL, Aurora, Ohio, USA) as a secondary antibody, and Rabbit peroxidase anti-Peroxidase (CAPPEL, Aurora, Ohio, USA) as a tertiary antibody, and the expression in trigeminal spinal nucleus in the medulla was compared.

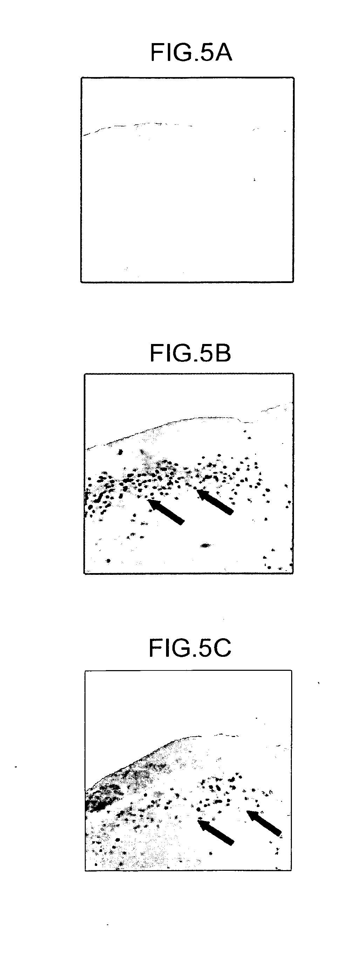

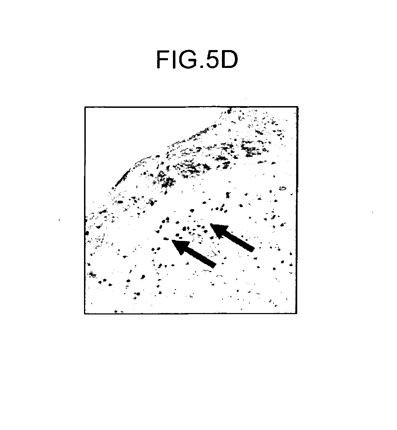

After removal of OCT compound, the sample was incubated in 0.3% hydrogen peroxide solution-80% methanol at room temperature for 1 hour to block endogenous enzyme activities. Subsequently, blocking was carried out with a blocking solution (3% Goat Serum-TBS) at room temperature for 1 hour, and the primary antibody was allowed to react with the sample at 4°C for 72 hours. After washing the sample, blocking was carried out with the blocking solution at room temperature for 1 hour, and the secondary antibody was allowed to react with the sample at room temperature for 1 hour. After washing the sample, blocking was carried out with the blocking solution at room temperature for 1 hour, and the tertiary antibody was allowed to react with the sample at room temperature for 1 hour. After sufficient washing, a DAB/NAS substrate solution (0.08 to 0.1 % nickel ammonium sulfate containing 0.04% DAB - 0.003% hydrogen peroxide-TBS) was added dropwise to the sample to allow coloring. The sections after the coloring were sufficiently washed and embedded in glycerol. The sample was observed with an upright microscope (Axioimager, manufactured by Zeiss). The results are shown in Fig. 5.

As a result, as shown in Fig. 5A, in trigeminal spinal subnucleus caudalis (the medullary dorsal horn) in C57BL/6 that was subjected to neither orthodontics of the regenerated tooth nor the dental pulp exposure treatment, expression of the c-Fos protein was not observed, but, as shown in Fig. 5B and Fig. 5C, in trigeminal spinal subnucleus caudalis (the medullary dorsal horn) in C57BL/6 that was subjected to orthodontics, a high level of expression of the c-Fos protein was induced 2 hours later, and the expression was maintained until 48 hours after the stimulation as shown in Fig. 5C. Further, as shown in Fig. 5D, in trigeminal spinal subnucleus caudalis (the medullary dorsal horn) in C57BL/6 that was subjected to the dental pulp exposure treatment of the regenerated tooth, a high level of expression of the c-Fos protein was induced 2 hours later. From these results, it was suggested that nerve fibers existing in the periodontal ligament region sense the compressive stimulation caused upon application of compressive stimulation by orthodontics to the regenerated tooth, and pain stimulation, and that the stimulation is transmitted to the central nervous system as in the case of a normal tooth.

Thus, it was revealed that, by using the restoration method of the present invention, a tooth-deficient area can be restored such that the regenerated tooth has hardness equivalent to a normal tooth, an ability to achieve normal occlusion, and responsiveness to stimuli equivalent.

The disclosure of Japanese Patent Application No. 2008-211870 is hereby incorporated by reference in its entirety.
All the literature, patent applications and technical standards described in the present specification are hereby incorporated by reference to the same extent as in cases where each literature, patent application or technical standard is concretely and individually described to be incorporated by reference.

## Claims

1. A method of producing a restorative material used to restore a tooth-deficient area in an oral cavity, the method comprising:
positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other;
culturing the first and second cell masses to form a reconstructed tooth germ or tooth; and
confirming directionality of the reconstructed tooth germ or tooth formed by the culturing so as to enable the reconstructed tooth germ or tooth to be embedded in the tooth-deficient area such that a tip of the tooth faces an interior of the oral cavity,
the tooth germ or tooth whose directionality has been confirmed being used as a restorative material to obtain an equivalent of a missing tooth in the tooth-deficient area.

2. The method of producing a restorative material according to claim 1, wherein the equivalent of a missing tooth has a Knoop hardness of enamel of 300 to 600 KHN and a Knoop hardness of dentin of 60 to 120 KHN.

3. The method of producing a restorative material according to claim 1 or 2, wherein the culturing is organ culturing and the restorative material comprises a reconstructed tooth germ or tooth formed by the organ culturing and a support carrier.

4. The method of producing a restorative material according to any one of claims 1 to 3, wherein both of the mesenchymal cells and the epithelial cells are derived from a tooth germ.

5. The method of producing a restorative material according to any one of claims 1 to 4, wherein each of the first cell mass and the second cell mass consists of a single cell.

6. The method of producing a restorative material according to any one of claims 1 to 5, wherein the support carrier is at least one selected from the group consisting of collagen, agarose gel, carboxymethyl cellulose, gelatin, agar, hydrogel, elastin, fibrin, fibronectin, laminin, an extracellular matrix mixture, polyglycolic acid (PGA), polylactic acid (PLA), lactic acid/glycolic acid copolymer (PLGA), Cellmatrix (trade name), Mebiol Gel (trade name) and Matrigel (trade name).

7. A method of restoring a tooth-deficient area in an oral cavity, the method comprising:
positioning, in a support carrier, a first cell mass formed from either mesenchymal cells or epithelial cells and a second cell mass formed from the other of the mesenchymal cells or epithelial cells, one of the mesenchymal cells or epithelial cells being derived from a tooth germ and the first and second cell masses being not mixed with each other but made to closely contact each other;
culturing the first and second cell masses to form a reconstructed tooth germ or tooth; and
embedding the reconstructed tooth germ or tooth in the tooth-deficient area.

8. The method of restoring a tooth-deficient area according to claim 7, wherein both of the mesenchymal cells and the epithelial cells are derived from a tooth germ.

9. The method of restoring a tooth-deficient area according to claim 7 or 8, wherein each of the first cell mass and the second cell mass consists of a single cell.

10. The method of restoring a tooth-deficient area according to any one of claims 7 to 9, wherein the support carrier is at least one selected from the group consisting of collagen, agarose gel, carboxymethyl cellulose, gelatin, agar, hydrogel, elastin, fibrin, fibronectin, laminin, an extracellular matrix mixture, polyglycolic acid (PGA), polylactic acid (PLA), lactic acid/glycolic acid copolymer (PLGA), Cellmatrix (trade name), Mebiol Gel (trade name) and Matrigel (trade name).
